# EUROPÄISCHE PATENTSCHRIFT

(11) **EP 0 829 720 B1**
(45) Veröffentlichungstag und Bekanntmachung des Hinweises auf die Patenterteilung: **17.01.2001**
(21) Anmeldenummer: 97115880.3
(22) Anmeldetag: 12.09.1997
(51) Int. Cl.: G01N 33/34, G01N 3/32, G01N 3/20

(54) **Vorrichtung zur Messung der Biegesteifigkeit von bewegtem blattförmigem Material**
Device for measuring the bending stiffness of a moving sheet
Appareil de mesure de la rigidité à la fléxion d'une bande en déplacement

(30) Priorität: 17.09.1996 DE 19637808
(43) Veröffentlichungstag der Anmeldung: 18.03.1998
(73) Patentinhaber: HONEYWELL AG, 63067 Offenbach am Main (DE)
(72) Erfinder: Syré, Hans-Richard, Dipl.-Ing., 56566 Neuwied (DE)
(74) Vertreter: Herzbach, Dieter, Dipl.-Ing.

(56) Entgegenhaltungen:
- EP-A- 0 325 542
- EP-A- 0 536 065
- NO-A- 68 924
- US-A- 4 291 577
- US-A- 5 171 403
- US-A- 5 470 005

## Beschreibung

Die vorliegende Erfindung betrifft eine Vorrichtung nach dem Gattungsbegriff des Patentanspruches 1.

Insbesondere bei der Herstellung von Karton ist die Messung der Biegesteifigkeit des Materials von besonderer Bedeutung, um diese während der Herstellung konstant zu halten bzw. bei Abweichungen direkt in den Prozeß (On-line) eingreifen zu können. Die Überwachung und Regelung der Biegesteifigkeit ist wichtig für die spätere Verarbeitung und den Gebrauch des Materials. Da Kartonmaschinen heutzutage Arbeitsgeschwindigkeiten von bis zu 500 m/min aufweisen, müssen Toleranzüberschreitungen früh genug erkannt werden und entsprechende Prozeßeingriffe vorgenommen werden.

Bei der Labormessung kann das Meßgut Karton als ein Biegebalken aufgefaßt werden. In der DIN Norm 53121 gibt es neben dem Zweipunktverfahren mit einer einseitigen Einspannung und einer Belastung am freien Ende noch das Dreipunktverfahren, bei dem zwei Auflager die zu messende Probe unterstützen und eine Kraft von oben wirksam wird. Diese Kraft wird so lange erhöht, bis eine definierte Durchbiegung erreicht ist. Die dann wirksame Kraft wird gemessen und der Wert wird in eine Formel eingesetzt, um die spezifische Biegesteifigkeit zu errechnen.

Bei einer laufenden Materialbahn bewegt sich das Meßgut durch eine ähnliche Anordnung. Der Unterschied zur Labormethode ist die Lagerung des Meßgutes auf zwei Rollen. Die von oben wirkende Kraft wird ebenfalls über eine Rolle auf das Meßgut gebracht. Diese Kraft wird durch eine Kraftmeßdose gemessen, wobei die Kraftmeßdose durch eine entsprechende Anordnung von Dehnungsmeßstreifen auf induktiver oder kapazitiver Basis, durch piezokeramische Elemente bzw. durch entsprechend ausgeformte Halbleiterbauelemente realisiert werden kann. Die Lagerung der Kraftmeßdose erfolgt an einem festen Auflager und die von der Kraftmeßdose aufgenommene Kraft wird in ein analoges elektrisches Signal umgeformt, das in einer entsprechende Auswerteelektronik ausgewertet wird. Die Auslenkung der Materialbahn erfolgt über einen Aktuator, der wahlweise zwischen dem festen Auflager und der Kraftmeßdose bzw. zwischen der Kraftmeßdose und der Rolle angeordnet sein kann.

Anders als im Laborversuch treten im vorliegenden Fall zusätzliche Kräfte auf, die durch die Bahnspannung verursacht werden. Wertet man daher das Signal der Kraftmeßdose alleine aus, so ist nicht erkennbar, ob die gemessene Kraft durch die Bahnspannung, die Biegesteifigkeit oder durch beide hervorgerufen wird.

Aus der EP 0 541 518 B1 und der US-A-5 171 403 sind gattungsgemäße Vorrichtungen bekannt, bei denen ein Materialbogen über eine kreisförmige Stützvorrichtung geführt wird, eine in der Mitte der Stützvorrichtung angeordnete Auslenkvorrichtung die Materialbahn auslenkt und eine Kraftmeßeinrichtung die von der Materialbahn auf die Stützvorrichtung ausgeübte Kraft mißt. Um die Biegesteifigkeit korrekt zu berechnen, müssen bei der EP 0 541 518 B1 einem Computer noch weitere Größen, u.a. die Bahnspannung zur Korrektur eingegeben werden. Die US-A-5 171 403 verwendet eine Betätigungseinrichtung, um die Auslenkvorrichtung um unterschiedliche Beträge auszulenken, wobei die hierbei gemessenen Kräfte in die Berechnung der Biegesteifigkeit einfließen. Ferner ist aus der EP 0 325 542 A3 eine Vorrichtung bekannt, bei der die Stärke einer Materialbahn ermittelt wird, indem die zur Anbringung von Perforierungen benötigte Kraft gemessen wird. Schließlich offenbart die US-A-5 470 005 eine Einrichtung zur Messung der Spannung einer Materialbahn, bei der eine Rolle in eine Vibrationsbewegung versetzt wird und eine an anderer Stelle in der Bahn angeordnete Kraftmeßeinrichtung die Bahnspannung mißt.

Aufgabe der vorliegenden Erfindung ist es, eine Vorrichtung anzugeben, die auf einfache Weise die Biegesteifigkeit einer Materialbahn zu ermitteln gestattet. Die Lösung dieser Aufgabe gelingt gemäß den kennzeichnenden Merkmalen des Patentanspruches 1. Weitere vorteilhafte Ausgestaltungen der erfindungsgemäßen Vorrichtung sind den abhängigen Ansprüchen entnehmbar.

Anhand der beiliegenden Zeichnungen sei im folgenden die Erfindung näher erläutert. Es zeigen:
- Fig. 1: eine Anordnung zur Ermittlung der Biegesteifigkeit in Längsrichtung der Materialbahn;
- Fig. 2: eine entsprechende Anordnung zur Ermittlung der Biegesteifigkeit in Querrichtung der Materialbahn;
- Fig. 3: eine erfindungsgemäße Anordnung;
- Fig. 4: weitere Möglichkeiten zur Erzielung einer periodischen Auslenkung der Materialbahn;
- Fig. 5: ein Signaldiagramm zur Erläuterung der Wirkungsweise der erfindungsgemäßen Vorrichtung; und
- Fig. 6: ein vektorielles Kräftediagramm zur Erläuterung der Erfindung.

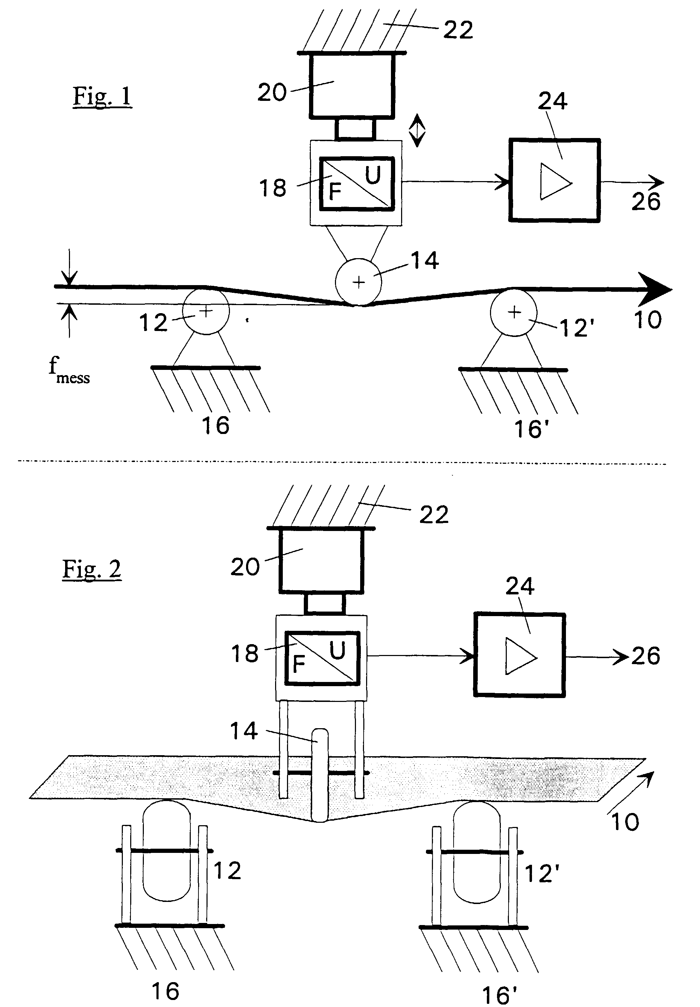

Gemäß dem Stand der Technik wird in Fig. 1 eine blattförmige Materialbahn 10, deren Biegesteifigkeit zu ermitteln ist, über Stützvorrichtungen in Form von Umlenkrollen 12 und 12' geführt. In der Mitte zwischen den Umlenkrollen 12 und 12' befindet sich eine Auslenkvorrichtung ebenfalls in Form einer Umlenkrolle 14. Während die Umlenkrollen 12 und 12' sich unmittelbar an ortsfesten Auflagern 16, 16' abstützen, ist die Umlenkrolle 14 über eine Kraftmeßdose 18 und einen Aktuator 20 an einem ortsfesten Auflager 22 abgestützt. Eine an die Kraftmeßdose 18 angeschlossene elektronische Auswerteeinrichtung 24 liefert an ihrem Ausgang 26 ein die Biegesteifigkeit des Materials kennzeichnendes Signal.

Als Aktuatoren 20 kommen alle mechanischen Konstruktionen in Frage, die eine Bewegung in Richtung zu der Materialbahn 10 ausführen können, z.B. Exzenter/Stößel- und Pleuel/Kurbel-Anordnungen. Gut einsetzbar sind auch pneumatische Kurzhubzylinder.Ferner können elektromechanische Aktuatoren, wie Spule/Stößel-Anordnungen sowie Piezo-Aktuatoren eingesetzt werden.

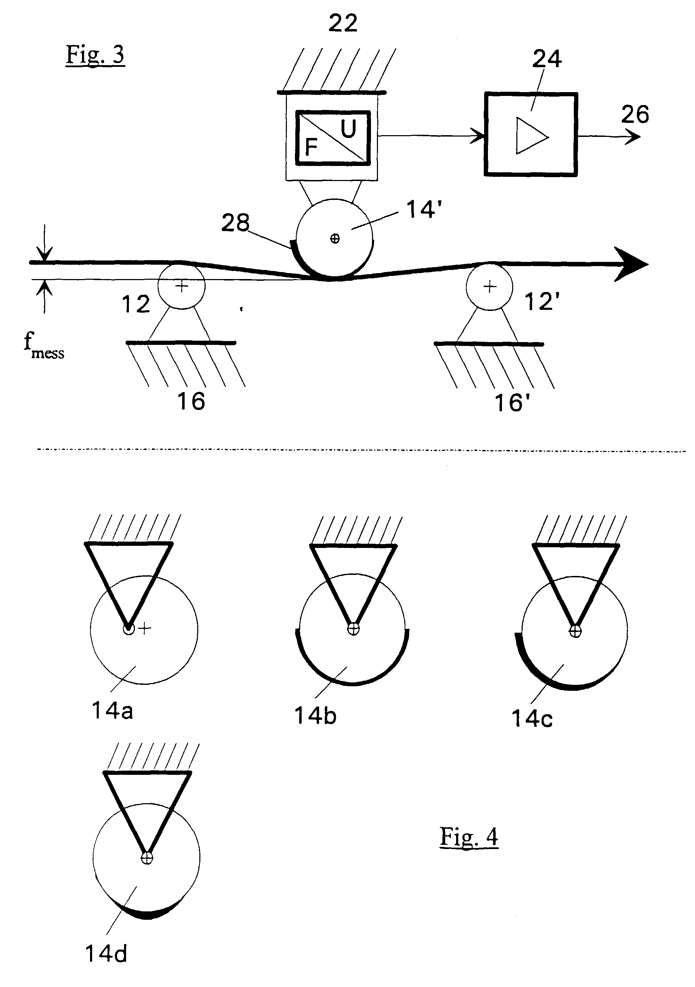

Fig. 2 zeigt eine Fig. 1 in ihren Komponenten entsprechende Vorrichtung, wobei sich die Materialbahn 10 in Richtung des eingezeichneten Pfeiles bewegt und die Umlenkrollen 12, 12' und 14 quer zur Materialbahn angeordnet sind. Wenn eine Materialbahn eine hohe anisotrope Festigkeitsverteilung aufweist, so kann es erforderlich sein, die Biegesteifigkeit sowohl in Quer- als auch in Längsrichtung der Materialbahn zu messen. Die gleiche Maßnahme wurde schon im Stand der Technik empfohlen

Im Gegensatz zeigt Fig. 3 eine erfindungsgemäße Vorrichtung. Die Vorrichtung entspricht im wesentlichen derjenigen in Fig 1, mit der Ausnahme, daß der Aktuator 20 entfallen ist und dafür die Umlenkrolle 14' mit einem Auftrag 28 versehen ist, der sich über einen Teil des Umfangs der Umlenkrolle 14' erstreckt. Die Umlenkrolle 14' wird hierbei in einfacher Weise durch Kontakt mit der Materialbahn an der Materialbahn 10 gedreht und erteilt hierbei der Materialbahn 10 eine periodische Auslenkung.

Andere Möglichkeiten der Ausgestaltung der Umlenkrolle 14 sind in Fig. 4 dargestellt. Die am einfachsten zu realisierende Lösung ist die außermittige Lagerung der Umlenkrolle 14a.

Hierdurch wird eine sinusförmige Änderung der Auslenkung der Materialbahn hervorgerufen. Ferner kann die Umlenkrolle 14b bei Lagerung im Zentrum über die Hälfte des Umfanges einen leicht erhöhten Radius aufweisen. Die Umlenkrolle 14c weist einen Auftrag in Form einer archimedischen Spirale auf, wobei sie wieder im Zentrum gelagert ist. Schließlich kann eine Umlenkrolle 14d mit einem Auftrag in beliebiger Kurvenform versehen sein, wobei aber immer darauf zu achten ist, daß diese Kurvenformen Kraftverläufe erzeugen, die gut durch analytische bzw, numerische Methoden zu analysieren und zu berechnen sind.

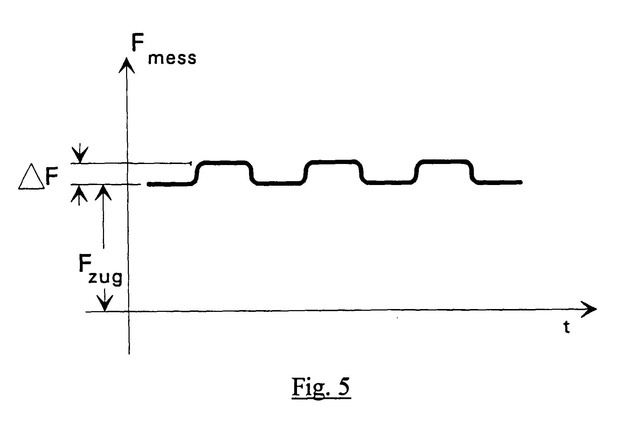

Fig. 5 zeigt ein Diagramm der gemessenen Kraft über der Zeit für den Fall, wo ein Exzenterrad 14b verwendet wird gemäß Fig. 4, das über die Hälfte des Umfangs eine geringe Radiusvergrößerung aufweist. Hierdurch überlagert sich der konstant wirkenden Zugkraft F_{zug} eine Kraft delta F, die durch den erhöhten Sektor des Exzenterrades hervorgerufen wird. Durch Auswertung dieser, der konstanten Kraft aufmodulierten Kraft, kann die Biegesteifigkeit des Materials ermittelt werden.

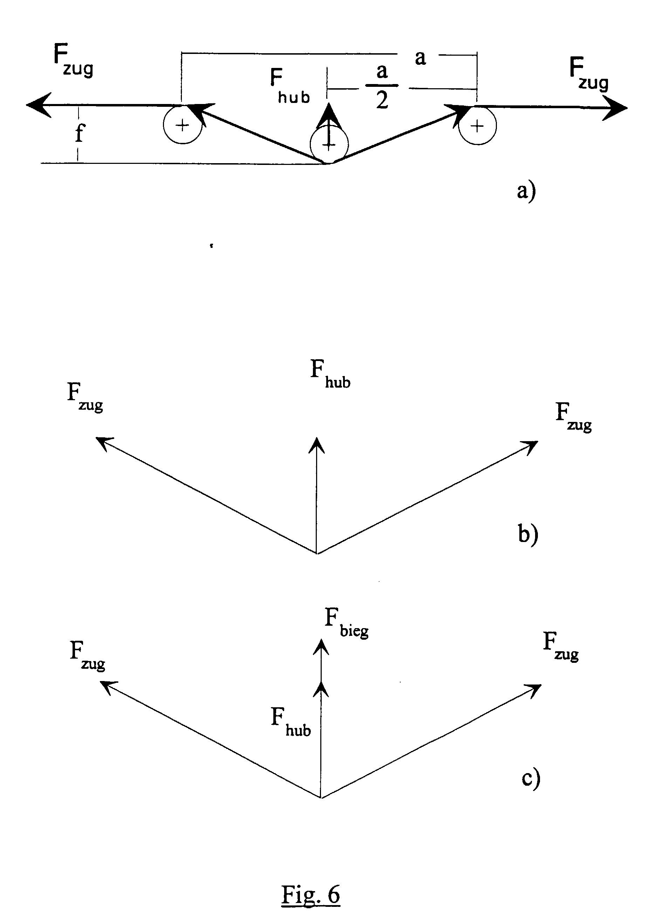

Fig. 6 zeigt ein vektorielles Kräftediagramm, wobei Fig. 6a eine Anordnung entsprechend der Vorrichtung gemäß Fig. 1 zeigt. Im Gegensatz zum Laborversuch treten im vorliegenden Fall zusätzliche Kräfte auf, die durch die Bahnspannung verursacht werden, was in Fig. 6b dargestellt ist. Im Falle einer Bahn ohne Biegesteifigkeit kann bei bekannten Zugkräften F_{zug} die Hubkraft F_{hub} graphisch wie rechnerisch ermittelt werden.

Im realen Fall, wo die Materialbahn eine Biegesteifigkeit aufweist, wirkt zusätzlich zur Hubkraft noch eine Biegekraft F_{bieg}, was in Fig. 6c dargestellt ist. Beide Kräfte wirken in dieselbe Richtung und sind zunächst als Einzelkräfte nicht meßbar. Durch die erfindungsgemäß getroffenen Maßnahmen kann der Anteil der Kraft ermittelt werden, der durch die Biegesteifigkeit hervorgerufen wird. Dies wird allgemein dadurch erreicht, daß die Eintauchtiefe der Umlenkrolle 14 periodisch verändert wird. Wird die Änderung der Eintauchtiefe so klein gewählt, daß die gesamte Weglängenänderung vernachlässigbar klein ist, so ist damit auch die Änderung der Zugkräfte F_{zug} zu vernachlässigen. Die Änderung der Kraft in der Bahneintauchrichtung ist dann nur noch auf die Biegesteifigkeit zurückzuführen.

## Patentansprüche

1. Vorrichtung zur Messung der Biegesteifigkeit einer bewegten blattförmigen Materialbahn (10), insbesondere von Karton, die über zwei beabstandete Stützvorrichtungen (12,12') geführt und durch eine dazwischen angeordnete Auslenkvorrichtung (14) senkrecht zur Materialbewegungsrichtung um einen vorbestimmten Betrag ausgelenkt wird, wobei eine Kraftmeßeinrichtung (18), die dem vorbestimmten Betrag zugeordnete Auslenkkraft mißt und diese Auslenkkraft der Biegesteifigkeit zuordnet, wobei Mittel vorgesehen sind, um die Auslenkvorrichtung (14) um unterschiedliche Beträge auszulenken, **dadurch gekennzeichnet,** daß die Auslenkvorrichtung (14) aus einer exzentrisch gelagerten Auslenkrolle (14a) bzw. aus einer über einen Teil ihres Umfanges mit einem Auftrag versehenen Auslenkrolle (14', 14b-d) besteht, die in Kontakt mit der bewegten Materialbahn steht, damit eine periodische Auslenkung der Materialbahn ermittellt wird.

2. Vorrichtung nach Anspruch 1, **dadurch gekennzeichnet,** daß die Stützvorrichtung durch zwei Stützrollen (12,12') vorgegeben ist, zwischen denen die Auslenkrolle (14) angeordnet ist.

3. Vorrichtung nach Anspruch 2, **dadurch gekenzeichnet,** daß die Stützrollen (12,12') und die Auslenkrolle (14) in Längsrichtung der bewegten blattförmigen Materialbahn (10) hintereinander angeordnet sind

4. Vorrichtung nach Anspruch 2, **dadurch gekennzeichnet,** daß die Stützrollen (12,12') und die Auslenkrolle (14a) in Querrichtung der bewegten blattförmigen Materialbahn (10) nebeneinander angeordnet sind.

5. Vorrichtung nach Anspruch 2, **dadurch gekennzeichnet,** daß die Stützrollen (12,12') und die Auslenkrolle (14) sowohl in Längsrichtung, als auch in Querrichtung der bewegten blattförmigen Materialbahn (10) hintereinander und nebeneinander angeordnet sind.

6. Vorrichtung nach einem der Ansprüche 1 bis 5, **gekennzeichnet durch** eine elektronische Auswerteeinrichtung (24), die an die Kraft-Meßeinrichtung (18) angeschlossen ist und das der periodischen Auslenkung zugeordnete Signal aus dem Gesamtsignal extrahiert.

## Claims

1. Device for measuring the bending stiffness of a moving sheet material web (10), especially of board, which is led over two spaced-apart supporting devices (12, 12') and is deflected by a predetermined amount perpendicular to the direction of movement of the material by a deflection device (14) arranged between the said supporting devices (12, 12'), a force measuring device (18) which measures the deflection force associated with the predetermined amount and assigns this deflection force to the bending stiffness, and means for deflecting the deflection device (14) by different amounts, being provided, characterized in that the deflection device (14) comprises an eccentrically mounted deflection roller (14a) or a deflection roller (14', 14b-d) which is provided with a coating over part of its circumference and is in contact with the moving material web in order that a periodic deflection of the material web is determined.

2. Device according to Claim 1, characterized in that the supporting device is provided by two supporting rollers (12, 12'), between which the deflection roller (14) is arranged.

3. Device according to Claim 2, characterized in that the supporting rollers (12, 12') and the deflection roller (14) are arranged after one another in the longitudinal direction of the moving sheet material web (10).

4. Device according to Claim 2, characterized in that the supporting rollers (12, 12') and the deflection roller (14) are arranged beside one another in the transverse direction of the moving sheet material web (10).

5. Device according to Claim 2, characterized in that the supporting rollers (12, 12') and the deflection roller (14) are arranged both after one another in the longitudinal direction and beside one another in the transverse direction of the moving sheet material web (10).

6. Device according to one of Claims 1 to 5, characterized by an electronic evaluation device (24), which is connected to the force measuring device (18) and which extracts the signal associated with the periodic deflection from the total signal.

## Revendications

1. Appareil de mesure de la rigidité en flexion d'une bande de matériau (10) en feuilles en mouvement, en particulier de carton, qui est guidée sur deux appareils de support espacés (12, 12') et qui est déviée d'une grandeur prédéterminée par un appareil de déviation intermédiaire (14) perpendiculairement au sens de déplacement du matériau, un dispositif de mesure de force (18) mesurant la force de déviation associée à la grandeur prédéterminée et associant cette force de déviation à la rigidité en flexion, des moyens étant prévus pour dévier l'appareil de déviation (14) suivant des grandeurs différentes, caractérisé en ce que l'appareil de déviation (14) se compose d'un rouleau de déviation (14a) monté excentrique ou d'un rouleau de déviation (14', 14b-d) pourvu d'un revêtement sur une partie de sa périphérie, qui est en contact avec la bande de matériau en mouvement, afin de déterminer une déviation périodique de la bande de matériau.

2. Appareil selon la revendication 1, caractérisé en ce que l'appareil de support est prédéfini par deux rouleaux de support (12, 12') entre lesquels est disposé le rouleau de déviation (14).

3. Dispositif selon la revendication 2, caractérisé en ce que les rouleaux de support (12, 12') et le rouleau de déviation (14) sont disposés les uns derrière les autres dans la direction longitudinale de la bande de matériau (10) en feuilles en mouvement.

4. Appareil selon la revendication 2, caractérisé en ce que les rouleaux de support (12, 12') et le rouleau de déviation (14) sont disposés les uns à côtés des autres dans la direction transversale de la bande de matériau (10) en feuilles en mouvement.

5. Appareil selon la revendication 2, caractérisé en ce que les rouleaux de support (12, 12') et le rouleau de déviation (14) sont disposés les uns derrière les autres et les uns à côté des autres dans la direction longitudinale ainsi que dans la direction transversale de la bande de matériau (10) en feuilles en mouvement.

6. Appareil selon l'une quelconque des revendications 1 à 5, caractérisé par un dispositif d'évaluation électronique (24) qui est raccordé au dispositif de mesure de force (18) et qui extrait du signal complet le signal associé à la déviation périodique.
